# EUROPEAN PATENT SPECIFICATION

(11) **EP 0 874 624 B1**
(45) Date of publication and mention of the grant of the patent: **03.12.2003**
(21) Application number: 96941564.5
(22) Date of filing: 23.12.1996
(51) Int. Cl.: A61K 31/045, A61K 47/48, A61K 31/70, A61K 38/08, C07F 9/117, C07K 7/06

(54) **HYALURONIC ACID RECEPTORS BINDING AGENTS AND THEIR USE FOR TREATING TUMORS OR RESTENOSIS**
HYALURONSÄUREREZEPTOREN BINDENDE STOFFE UND DEREN VERWENDUNG ZUR BEHANDLUNG VON TUMOREN UND RESTENOSE
LIGANDS DES RECEPTEURS A L'ACIDE HYALURONIQUE ET LEUR UTILISATION DANS LE TRAITEMENT DES TUMEURS ET DE LA RESTENOSE

(30) Priority: 27.12.1995 CA 2166155
(43) Date of publication of application: 04.11.1998
(73) Proprietor: JAGOTEC AG, 4132 Muttenz (CH)
(72) Inventor: TURLEY, Eva, Toronto, Ontario M8V 3Y1 (CA)
(74) Representative: Bassil, Nicholas Charles
(86) International application number: CA9600870
(87) International publication number: WO97024111

(56) References cited:
- EP-A- 0 656 213
- WO-A-93/21312
- WO-A-96/06622
- EMBO JOURNAL, vol. 13, no. 2, 1994, pages 286-296, XP000647316 BAIHUA YANG ET AL.: "Identification of a common hyaluronan binding motif ..." cited in the application

## Description

### FIELD OF THE INVENTION

This invention relates to novel binding agents suitable for use to treat diseases and conditions treatable by receptor mediated treatment and the use of such agents to bind with receptors at the sites in the body of a mammal in need of treatment and, in one application, finds use in the treatment of malignant tumours in humans.

### BACKGROUND OF THE INVENTION

WO 91/04058 owned by Hyal Pharmaceutical Corporation teaches the use of dosages of at least 10 mg. of forms of hyaluronic acid (HA) to transport effective amounts of medicines and/or therapeutic agents to sites in need of treatment in the human body, to penetrate the tissue at the sites in need of treatment, including scar tissue, through all membranes into the cells to be treated.

At page 25, line 17, the PCT Application teaches the additional benefit of using at least about 200 mg. of forms of hyaluronic acid (for example, sodium hyaluronate) in a dosage together with the medicine and/or therapeutic agent for reducing the side effects of the medicine and/or therapeutic agent when administered (such as gastro-intestinal distress, neurological abnormalities, depression, etc. normally associated with the medicine and/or therapeutic agent) even at elevated amounts greater than the usual accepted dosage amounts of the medicine and/or therapeutic agent when administered alone for example, an NSAID (non-steroidal anti-inflammatory agent).

WO 96/06622 owned by Hyal Pharmaceutical Corporation, teach the modulation of cellular activity of tissue and cells expressing a high affinity cell-surface receptor for hyaluronic acid by the use of forms of hyaluronic acid. These cell surface receptors comprise adhesion molecule ICAM-1, adhesion molecule CD44 and adhesion molecule HARLEC (Hyaluronic Acid [Hyaluronan] Receptors Liver Endothelial Cells) and regulatory molecule RHAMM (Receptor for HA Mediated Motility), for binding hyaluronan. HARLEC is expressed (produced and put on the cell surface) in liver endothelial cells. The administration of an effective amount of a form of hyaluronic acid to bind with the cell-surface receptors modulates cellular activity of tissues and/or cells expressing such high affinity cell-surface receptors for hyaluronic acid (for example, an adhesion or regulatory molecule) in the human body.

One of the reasons why the hyaluronic acid is able to be used to transport the medicine and/or therapeutic agent is its selective binding to the cell-surface receptors.

The body has at least 10²⁸ different combinations of amino acids (see *Identification of Recognition Sequences of Adhesion Molecules Using Phage Display Technology*, O'Neil, Karyn T., et al; Methods in Enzymology, Vol. 245, (1994), 370-386.) When the form of hyaluronic acid, sodium hyaluronate (HA) is put into the body as bait, only two combinations of amino acids from the at least 10²⁸ total combinations are combined with the HA. These two combinations are highly specific for HA.
1. The first involves the use of the amino acid sequence as follows:
   Single Letter Code: STMMSRSHKTRSHHV
   Three Letter Code: 
Ser-Thr-Met-Met-Ser-Arg-Ser-His-
Lys-Thr-Arg-Ser-His-His-Val
   The underlined portion is the basic amino acid motif for HA binding proteins (Rhamm, ICAM-1, CD44, HARLEC etc.). The underlined portion is the binding domain.
2. The second series of amino acid binding domains is found in a second amino acid sequence, namely:
   Single Letter Code: TMTRPHFHKRQLVLS
   Three Letter Code: 
Thr-Met-Thr-Arg-Pro-His-Phe-His-
Lys-Arg-Gln-Leu-Val-Leu-Ser
   This amino acid has a smaller binding domain. This binding domain is for the nucleus {nuclear}. This smaller binding domain also binds to the HA binding protein Rhamm which is the only HA receptor that translocates to the nucleus.

The letters each represent a different amino acid identified as follows in the enclosed table:

| The 20 Amino Acids in Proteins | | |
|---|---|---|
| | Three-Letter Code | Single Letter Code |
| Glycine | GLY | G |
| Alanine | ALA | A |
| Valine | VAL | V |
| Isoleucine | ILE | I |
| Leucine | LEU | L |
| Serine | SER | S |
| Threonine | THR | T |
| Proline | PRO | P |
| Aspartic acid | ASP | D |
| Glutamic acid | GLU | E |
| Lysine | LYS | K |
| Arginine | ARG | R |
| Asparagine | ASN | N |
| Glutamine | GLN | Q |
| Cysteine | CYS | C |
| Methionine | MET | M |
| Tryptophan | TRP | W |
| Phenylalanine | PHE | F |
| Tyrosine | TYR | Y |
| Histidine | HIS | H |

The evidence that the above motifs represent HA binding domains is found in the article entitled "*Identification of a common hyaluronan binding motif in the hyaluronan binding proteins RHAMM, CD44 and link protein* ", The EMBO Journal, Vol. 13, No. 2 (1994) pp. 286-296.

I have found, through tests, that another molecule PIP-2 (phosphatidylinositol-4,5-bis phosphate) of which phosphoinositol-4,5,-bis-phosphate is a portion) binds specifically to these same binding domains. PIP2 has the following structural formula: 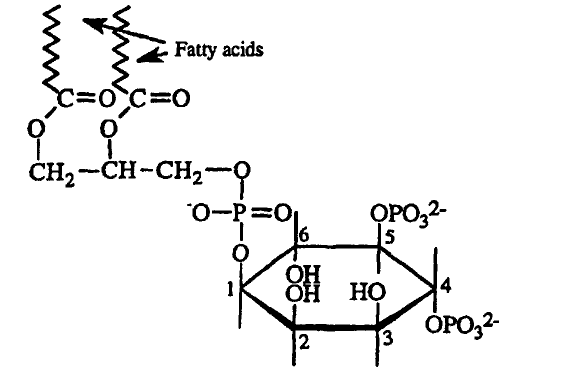 of which the portion below is the active portion:

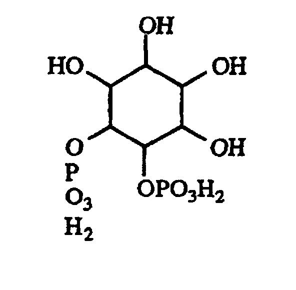

As is known, Rhamm binds with HA. In carrying out my tests, I discovered that Rhamm also binds with PIP2. If excess PIP2 is used, it displaces the HA. Rhamm is a very important receptor in that it becomes "upregulated" for tumourigenesis and restenosis and other instances of repair. Thus, PIP2 and HA are important in this regard. HA has been used successfully to transport medicines to the site of disease or conditions with its ability to selectively bind with cell surface receptors (including Rhamm). Thus, PIP2 can be used to treat the same conditions and diseases as has been treated with HA (either alone or with a medicine and/or therapeutic agent). As PIP2 is a smaller molecule, PIP2 or any compound that mimics PIP2 or HA and thus, any analogue, homologue, derivative, complex, etc. which mimics HA and PIP2 could be used effectively and more easily. These compounds could be new, synthetic or purified natural compounds (be found in nature in their unpurified form).

It is therefore an object of this invention to provide novel binding agents.

It is a further object of the invention to provide methods of treatment using the novel binding agents to treat diseases and conditions treatable by receptor mediated treatment to bind with receptors at the sites in the body of a mammal in need of treatment for example, in the treatment of malignant tumours and for example, the treatment of restenosis.

Further and other objects of the invention will be realized by those skilled in the art from the following summary of invention and detailed description of embodiments thereof.

### SUMMARY OF INVENTION

According to a first aspect of the invention, there is provided the use of a binding agent (other than hyaluronan, a pharmaceutically acceptable salt thereof or other such forms of hyaluronic acid) selected from the group consisting of:
(i) 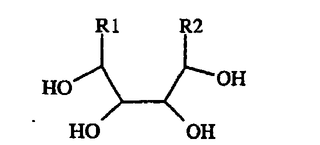 where either
   (a) R₁ and R₂ form a bridge forming a closed ring molecule; or
   (b) R₁ and R₂ are not joined and each of R₁ and R₂ is a lower alkyl and
(ii) a binding agent which includes phosphoinositol-4,5-bisphosphate as an active portion,
   in the manufacture of a medicament for the treatment of, or prevention of malignant tumours or restenosis in which the binding agent binds at the site of the disease or condition with at least one of the group of binding domains selected from:
   (a) RSHKTRSHH
      {*Single-Letter Code of Amino Acids}*
      also identified as: Arg-Ser-His-Lys-Thr-Arg-Ser-His-His {*Three-Letter Code of Amino Acids*}
      also encoded for by: CGC-TCG-CAC-AAG-ACC-AGG-TCG-CAC-CAC
   (b) RPHFHKR
      {*Single-Letter Code ofAmino Acids}*
      also identified as: Arg-Pro-His-Phe-His-Lys-Arg {*Three-Letter Code of Amino Acids*}
      also encoded for by:

CGG-CCC-CAC-TTC-CAC-AAG-CGG

The medicament may comprise an effective amount of the binding agent. The binding agent may be mixed with the medicine and/or therapeutic agent.

One such binding agent is the active portion phosphoinositol-4,5-bisphosphate of PIP2. The binding agent may be mixed with the medicine and/or therapeutic agent. Because the novel binding agents bind to the above domains which also bind hyaluronan, my invention can be applied for any receptor mediated treatment for which hyaluronan is useful. My invention relates to binding agents which replace hyaluronan. Thus, my invention can be used for restenosis prevention and other treatments in the same manner that hyaluronan is used. Thus, my invention can be used for the treatments and preventative therapies discussed in the following published and unpublished documents identified below including Canadian Patent No. 2,164,260 filed in the Canadian Patent Office on the 1st day of December, 1995 entitled "*Targeting of Dosages of Medicines and Therapeutic Agents*", and Canadian Patent No. 2,173,037 filed March 29, 1996 entitled "*Targeting of Dosages of Medicines and Therapeutic Agents and other Glycosaminoglycans (GAGS)*" and

### International Publication

WO 91/04058
WO 93/16732
WO 93/16733
WO 94/07505
WO 94/23725
WO 95/29683
WO 95/26193
WO 95/30423
WO 96/15845
WO 96/06622

With respect to each of the documents, the forms of hyaluronan may be substituted by for example, the other binding agents referred to herein and be used in like manner.

As an example, I have extracted from WO 91/04058 the following to illustrate just some of the uses:

### (i) at page 17, line 3 to page 18, line 16:

"Applicants have now discovered that combinations and formulations (for example an injectable formulation) can be provided for administration to a mammal for the treatment of a disease or condition, which combinations or formulations employ or incorporate as the case may be a therapeutically effective non-toxic amount of a medicinal and/or therapeutic agent to treat the disease or condition (for example a free radical scavenger (for example ascorbic acid (Vitamin C)), Vitamin C (for the treatment of mononucleosis), an anti-cancer agent, chemotherapeutic agent, anti-viral agents for example a nonionic surfactant, e.g. nonoxynol-9 [nonylphenoxy polyethoxy ethanol] found in Delfen™ contraceptive cream, and anionic surfactants (e.g. cetyl pyridinium chloride) and cationic surfactants (e.g. benzalkonium chloride), non-steroidal anti-inflammatory drugs (NSAID) for example indomethacin, naproxen and (+/-) tromethamine salt of ketorolac (sold under the trademark Toradol™) and steroidal anti-inflammatory drugs, anti-fungal agent, detoxifying agents (for example for administration rectally in an enema), analgesic, bronchodilator, anti-bacterial agent, antibiotics, drugs for the treatment of vascular ischemia (for example diabetes and Berger's disease), anti-body monoclonal agent, minoxidil for topical application for hair growth, diuretics (for example furosemide (sold under the trademark Lasix™)), immunosuppressants (for example cyclosporins), lymphokynes (such as interleukin - 2 and the like), alpha-and-β-interferon and the like) administered with, or carried in, an amount of hyaluronic acid and/or salts thereof (for example the sodium salt) and/or homologues, analogues, derivatives, complexes, esters, fragments, and/or sub units of hyaluronic acid (preferably hyaluronic acid and salts thereof) sufficient to facilitate the agent's penetration through the tissue (including scar tissue), at the site to be treated through the cell membranes into the individual cells to be treated. When such combinations and formulations are administered to patients suffering from the disease or condition, the disease or condition is unexpectedly improved.

The formulation can be administered among other methods, intravenously, intra arterially, intraperitoneally, intrapleurally, transdermally, on the skin (topically), rectally, orally or by direct injection (for example into a tumor, into an abscess or similar disease focus) or put on a patch to be secured to the skin of the patient. The hyaluronic acid and/or salts thereof and the agent can be administered separately but are administered in sufficient amounts and in an immediate time sequence or interval (preferably concurrently and more preferably simultaneously), preferably at the identical site (e.g. one given intravenously and the other "piggy backed"), to treat the disease or condition."

### (ii) at page 25, line 18 to page 26, line 14:

"Thus and according to another aspect of the invention when an NSAID for example indomethacin (dissolved in n-methyl glucamine) or other NSAID is administered with greater than 200mg hyaluronic acid for 1-2 mg/kg body weight of the NSAID (in one instance indomethacin and NMG), no major toxic side effects occur such as gastro-intestinal distress, neurological abnormalities, depression, etc., even at elevated amounts of indomethacin (if necessary). If the amount of hyaluronic acid is decreased below that amount, the usual side effects may begin to reoccur. In addition, the responses that have been observed are superior when the NSAID (for example Indocid™) is combined with hyaluronic acid demonstrating clearly that the combination is now "targeting" to the pathological tissue even when administered by the systemic intravenous route. Thus, it has been observed that patients with neoplastic diseases when receiving in addition to other chemicals (for example ascorbic acid [Vitamin C], phloretin and anti-cancer drugs), 50 - 200 mg NSAID - hyaluronic acid (sodium hyaluronate) (for example indomethacin and hyaluronic acid) experience dramatic relief of pain immediately. This is followed within a short period of time by a resolution and resorbtion of neoplastic lesions with an improvement of pulmonary, and liver function if there is tumor present in these organs. Thus the dead tumor material and the debris and tumor toxins appear to be better eliminated by the body through the action of the macrophages whose activity is enhanced by the addition of the NSAID (or a steroidal anti-inflammatory drug) administered with hyaluronic acid (or salt or other form thereof). Thus Applicants believe that the addition of the NSAID for example with hyaluronic acid (sodium hyaluronate) deblocks the macrophages by preventing enzymatic production of prostaglandin synthetase which blocks macrophage functioning. Thus the hyaluronic acid (and salt and other forms) not only enhance the activity of the NSAID but also reduce any side effects and toxicity that is associated with the use of the prostaglandin synthesis inhibitors.

Examples of agents suitable for use. as chemotherapeutic agents are novantrone (Mitoxantrone), Methotrexate, 5-FU (5-Fluouracil), carboplatinum, methyl CCNU administered orally and Mitomycin C."

### iii) at page 26, lines 32 to 37:

"The hyaluronic acid and salts thereof may be utilized at varying doses - 10 to 1000 mg/70 kg person with the optimal doses tending to range between 50 and 350 mg/70 kg individual. As there is no toxicity, the hyaluronic acid can obviously be administered in a dose excess (for example 3000 mg/70 kg individual) without any adverse effects."

### (iv) at page 29, line 27 to page 33, line 31:

"One form of hyaluronic acid and /or salts thereof (for example sodium salt) and homologues, analogues, derivatives, complexes, esters, fragments, and sub units of hyaluronic acid, preferably hyaluronic acid and salts and thereof suitable for use with Applicant's invention is a fraction supplied by Sterivet Laboratories Limited. One such fraction is a 15 ml vial of Sodium hyaluronate 20mg/ml (300mg/vial - Lot 2F3). The sodium hyaluronate fraction is a 2% solution with a mean average molecular weight of about 225,000. The fraction also contains water q.s. which is triple distilled and sterile in accordance with the U.S.P. for injection formulations. The vials of hyaluronic acid and/or salts thereof may be carried in a Type 1 borosilicate glass vial closed by a butyl stopper which does not react with the contents of the vial."

The fraction of hyaluronic acid and/or salts thereof (for example sodium salt) and homologues, analogues, derivatives, complexes, esters, fragments, and sub units of hyaluronic acid, preferably hyaluronic acid and salts thereof may comprise hyaluronic acid and/or salts thereof having the following characteristics:
a purified, substantially pyrogen-free fraction of hyaluronic acid obtained from a natural source having at least one characteristic selected from the group consisting of the following:
   i) a molecular weight within the range of 150,000-225,000;
   ii) less than about 1.25% sulphated mucopolysaccharides on a total weight basis;
   iii) less than about 0.6% protein on a total weight basis;
   iv) less than about 150 ppm iron on a total weight basis;
   v) less than about 15 ppm lead on a total weight basis;
   vi) less than 0.0025% glucosamine;
   vii) less than 0.025% glucuronic acid;
   viii) less than 0.025% N-acetylglucosamine;
   ix) less than 0.0025% amino acids;
   x) a UV extinction coefficient at 257 nm of less than about 0.275;
   xi) a UV extinction coefficient at 280 nm of less than about 0.25; and
   xii) a pH within the range of 7.3-7.9. Preferably the hyaluronic acid is mixed with water and the fraction of hyaluronic acid fraction has a mean average molecular weight within the range of 150,000-225,000. More preferably the fraction of hyaluronic acid comprises at least one characteristic selected from the group consisting of the following characteristics:

   i) less than about 1% sulphated mucopolysaccharides on a total weight basis;
   ii) less than about 0.4% protein on a total weight basis;
   iii) less than about 100 ppm iron on a total weight basis;
   iv) less than about 10 ppm lead on a total weight basis;
   v) less than 0.00166% glucosamine;
   vi) less than 0.0166% glucuronic acid;
   vii) less than 0.0166% N-acetylglucosamine;
   viii) less than 0.00166% amino acids;
   x) a UV extinction coefficient at 257 nm of less than about 0.23;
   xi) a UV extinction coefficient at 280 nm of less than 0.19; and
   xii) a pH within the range of 7.5-7.7

Other forms of hyaluronic acid and/or its salts, and homologues, derivatives, complexes, esters, fragments and sub units of hyaluronic acid may be chosen from other suppliers, for example those described in the prior art documents previously referred to. In addition Applicants have successfully employed sodium hyaluronate produced and supplied by LifeCore™ Biomedical, Inc. having the following specifications

| Characteristics | | | | | Specification | | | |
|---|---|---|---|---|---|---|---|---|
| Appearance | | | | | White to cream colored particles | | | |
| Odor | | | | | No perceptible odor | | | |
| Viscosity Average | | | | | < 750,000 Daltons | | | |
| Molecular Weight | | | | | | | | |
| UV/Vis Scan, 190-820nm | | | | | Matches reference scan | | | |
| OD, 260nm | | | | | < 0.25 OD units | | | |
| Hyaluronidase Sensitivity | | | | | Positive response | | | |
| IR Scan | | | | | Matches reference | | | |
| pH, 10mg/g solution | | | | | 6.2 - 7.8 | | | |
| Water | | | | | 8% maximum | | | |
| Protein | | | | | < 0.3 mcg/mg NaHy | | | |
| Acetate | | | | | < 10.0 mcg/mg NaHy | | | |
| Heavy Metals, maximum ppm | | | | | | | | |
| As | Cd | Cr | Co | Cu | Fe | Pb | Hg | Ni |
| 2.0 | 5.0 | 5.0 | 10.0 | 10.0 | 25.0 | 10.0 | 10.0 | 5.0 |
| Microbial Bioburden | | | | | None observed | | | |
| Endotoxin | | | | | < 0.07EU/mg NaHy | | | |
| Biological Safety Testing | | | | | Passes Rabbit Ocular | | | |
| | | | | | Toxicity Test | | | |

The following references teach hyaluronic acid, sources thereof and processes of the manufacture and recovery thereof.

United States Patent 4,141,973 teaches hyaluronic acid fractions (including sodium salts) having:
'(a) an average molecular weight greater than about 750,000, preferably greater than about 1,200,000 - that is, a limiting viscosity number greater than about 1400 cm³/g., and preferably greater than about 2000 cm³/g.;
(b) a protein content of less than 0.5% by weight;
(c) ultraviolet light absorbance of a 1% solution of sodium hyaluronate of less than 3.0 at 257 nanometers wavelength and less than 2.0 at 280 nanometers wavelength;
(d) a kinematic viscosity of a 1% solution of sodium hyaluronate in physiological buffer greater than about 1000 centistokes, preferably greater than 10,000 centistokes;
(e) a molar optical rotation of a 0.1 - 0.2% sodium hyaluronate solution in physiological buffer of less than - 11 X 10³ degree - cm²/mole (of disaccharide) measured at 220 nanometers;
(f) no significant cellular infiltration of the vitreous and anterior chamber, no flare in the aqueous humor, no haze or flare in the vitreous and no pathological changes to the cornea, lens, iris, retina, and choroid of the owl monkey eye when one milliliter of a 1% solution of sodium hyaluronate dissolved in physiological buffer is implanted in the vitreous replacing approximately one-half the existing liquid vitreous, said HUA being
(g) sterile and pyrogen free and
(h) non-antigenic.'

Canadian Letters Patent 1,205,031 (which refers to United States Patent 4,141,973 as prior art) refers to hyaluronic acid fractions having average molecular weights of from 50,000 to 100,000; 250,000 to 350,000; and 500,000 to 730,000 and discusses processes of their manufacture.

Where high molecular weight hyaluronic acid (or salts or other forms thereof) is used, it must be diluted to permit administration and ensure no intramuscular coagulation."

### (vi) and, at page 33, line 37 to page 35, line 30:

"Thus Applicant has combined hyaluronic acid (and sodium hyaluronate and/or other forms) with medicinal and/or therapeutic agents for the treatment of conditions and diseases with totally unexpected results:

### For Example

| Condition/Disease | Chemicals & Drugs |
|---|---|
| 1. Cancer, increasing activity of macrophages | free radical scavenger, superoxide dismutase, ascorbic acid(Vitamin C) anti-cancer drugs, NSAID, Chemotherapeutic Agents, detoxifying Agents (e.g. cholestyramine) |
| 1A. Reduction of swelling in brain of (DMSO)person suffering brain trauma | Dimethyl Sulfoxide |
| 2. Hair growth grow more hair when applied topically | minoxidil - combination - |
| 3. Herpes, canker sore, shingles | nonionic surfactants, e.g., nonoxynol-9 and anionic, (e.g. cetyl pyridinium chloride) and cationic (e.g. benzalkonium choride), surfactants |
| 4. Renal failure, cardiac insufficiency, hypertension, edema | diuretics - furosemide |
| 5. Infection, acne, mononucleosis | antibiotics, antibacterials, antimicrobials, etc., ascorbic acid and hyaluronic acid |
| 6. Transplants | cyclosporins |
| 7. Inflammation, elimination of tumor break down material | non-steroidal anti-inflammatories, NSAID e.g. (toxins and debris), diclofenac, |
| decreasing side effects, relief of pain (e.g. | indomethacin, piroxicam, ibuprofen, tromethamine |
| back pain) | salt of Ketorolac, naproxen, |
| 8. Detoxification | enema, detoxifying agent, peritoneal dialysis |
| 9. Bronchodilation | bronchodilators, e.g. beclo-methasone diproprionate (sodium cromoglycate although not specifically a broncho-dialator), theophylline |
| 10. Vascular ischemia | treat limbs in respect of diabetes, Berger's disease, etc. with suitable medicine e.g. Trental |
| 11. HIV (AIDS) | DMSO, Vitamin C, NSAID (e.g. indomethacin, naproxen, ketorolac tromethamine), interferon, Vibramycin™, (doxcycline), tetracycline |
| 12. Diabetes | insulin |
| 13. Post-menopause | estrogens replacement |
| 14. Prevention of topical | antimetabolites (e.g. infection sulfonamides) |
| 15. Reduction of swelling | DMSO |
| 16. Hypertension, cardiac | Calcium channel blockers e.g. insufficiency- Nifedipine β-Blockers e.g. atenolol, propranolol |
| 17. Prostaglandin Synthesis inhilition | acetylsalicylic acid |
| 18. Enhance oxygenation of tissue by perfusion fluid bathing the tissue (for transplantation purposes" | perfusate |

Thus, for example, in the prevention of restenosis, the smooth muscle cells do not migrate into, and the white cells (macrophages) do not infiltrate, the stenotic plaque after administration of an effective amount of the binding agent.

Suitable amounts of the binding agent which are expected to be therapeutic are between about .1 to about 1.5mg. of binding agent (for example, PIP2) per kilogram of body weight. Preferably about 0.2-1 mg. of binding agent (for example PIP2) per kilogram of body weight of the patient being treated is administered. This amount can be reduced or increased to larger dosage amounts and be used effectively. However, the binding agent would preferably not be administered in amounts which are toxic either to the cells or to the individual. In some cases, however, cells may die from administering the effective dosage amount of the binding agent causing beneficial effects to individuals even where the amount of the binding agent used is not considered toxic. The administration takes place as long as required.

PIP2, as mentioned above, is suitable for the purposes of the invention when used in dosages containing for example an amount of for example, between about .1 to about 1.5mg./kg of body weight such as 0.2-1 mg. of binding agent per kilogram of body weight. To enhance the beneficial effects (for example, therapeutic effects) of PIP2, PIP2 may be modified to reduce its fat solubility by for example, linking PIP2 to glucuronic acid or other suitable non-toxic hydrophilic agents thereby reducing PIP2's fat solubility. A reaction product may be prepared from the PIP2 and hydrophilic agent.

The PIP2 binding molecule may also itself be modified to substitute for the phosphate by other moieties such as sulphate, carbonate, nitrate, sulphite, nitrite or other suitable moiety.

It is also believed that the inositol ring in its entirety is not necessary to achieve the desired binding to the receptors. It appears that only a portion of the chair structure need be used comprising four carbon atoms to which are fixed the hydroxyl radicals. Thus, another molecule including those four carbons forming the chair configuration of the formula may be suitable. Thus, a compound of the structural formula

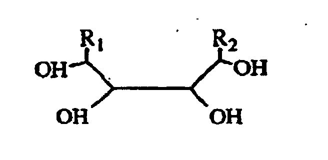

R₁ and R₂ may not be joined or R₁ and R₂ may form a bridge forming a closed ring molecule and where R₁ and R₂ may each be selected from lower alkyl or other suitable substituent may be suitable.

The antisense peptide for each of
(a) RSHKTRSHH
   *{Single-Letter Code of Amino Acids}* Arg-Ser-His-Lys-Thr-Arg-Ser-His-His *{Three-Letter Code of Amino Acids}*
(b) RPHFHKR
   *{Single-Letter Code of Amino Acids}* Arg-Pro-His-Phe-His-Lys-Arg *{Three-Letter Code of Amino Acids}*
   may also be suitable. These are readily and easily determined by persons skilled in the art. They can also be made by known methods as would be understood by persons skilled in the art. So may PIP2 mimetics be suitable as well as other hyaluronan mimetics (which are not forms of hyaluronan). The anti-sense peptides are as follows:
   - for (a), the anti-sense peptide is ASVFWSSVV, (another code: GCG-AGC-GTG-TTC-TGG-TCC-AGC-GTG-GTG);
   - for (b), the anti-sense peptide is AGVKVFA (another code: GCC-GGG-GTG-AAG-GTG-TTC-GCC).

The anti-sense peptides may be manufactured by any method as would be known by persons skilled in the art and are easily determined from the peptide. Some guidance may be found in:
(i) "*Identification of Recognition Sequences of Adhesion Molecules Using Phage Display Technofogy*", Methods in Enzymology, Vol. 245 at page 370, and
(ii) "*Molecular Cloning of a Novel Hyaluronan Receptor that Mediates Tumour Cell Motility*", The Journal of Cell Biology, Vol. 117, 1992 at page 1343.

Suitable amounts of the anti-sense peptides would be in the order of those previously described such as between about 1 to about 1.5 mg. per kilogram of body weight. These amounts may be reduced or increased to larger dosage amounts and be used effectively. Once again, the agent is not used which is toxic to the cells or to the individual. In some cases however, cells may die from administering the effective dosage amount of the anti-sense peptides as a binding agent causing beneficial effects to the individuals even where the amount of the binding agent used is not considered toxic. The administration takes place as long as required.

### BRIEF DESCRIPTION OF DRAWINGS

### Figure 1

Binding of Phage containing the sequence RSHKTRSHH to a biotinylated hyaluronan probe in the presence of (1) biotinylated hyaluronan only; (2) 5 µg/ml PIP2; (3) 50 µg/ml PIP2; (4) 250 µg/ml PIP2 and (5) 100 µg/ml unlabeled hyaluronan. These results show that PIP2 effectively inhibit the binding of hyaluronan to the above sequence. The ability of unlabeled hyaluronan to compete with biotinylated hyaluronan for this sequence confirms the specificity of the interaction between hyaluronan and the sequence.

### Figure 2

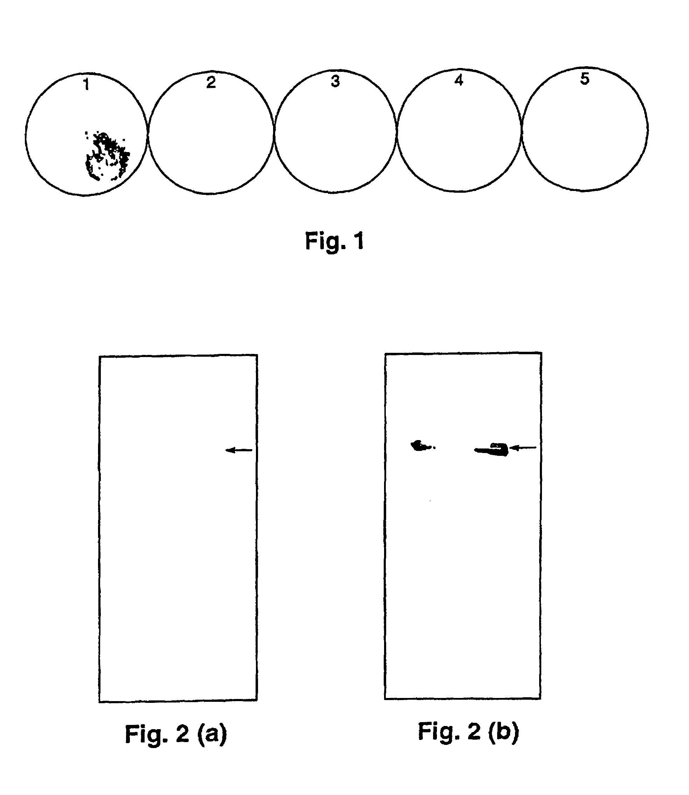

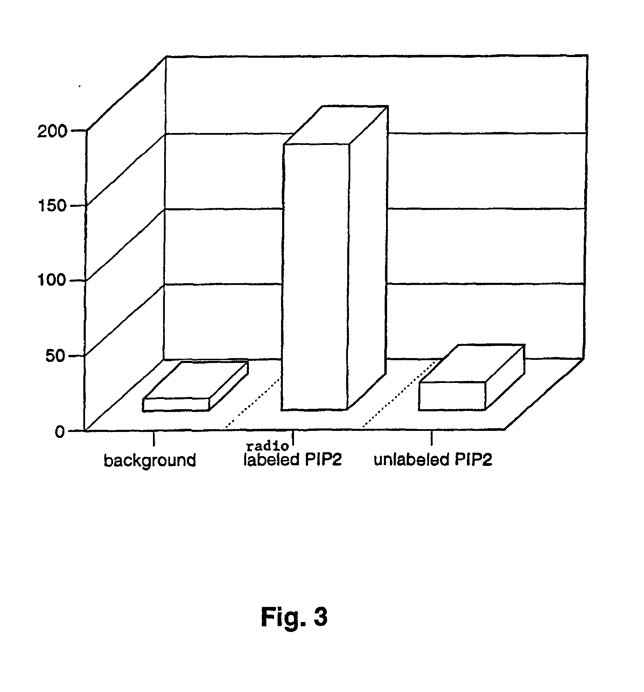

The ability of PIP2 to inhibit the binding of hyaluronan to RHAMM. (a) Biotinylated hyaluronan binds to RHAMM (see Yang, et. al., entitled "*Identification of a common link protein*" (The EMBO Journal, Vol. 13, No. 2, pp. 286-296, 1994) for details and controls which reference is incorporated herein by reference); (b) 250 µg/ml of PIP2 essentially abolishes the ability of hyaluronan to bind to RHAMM. Since the sequence described in Figure 1 RSHKTRSHH, contains the hyaluronan binding motif of basic amino acid (7 intervening amino acids) Basic amino acid (see Yang, et. al., above for details) and this binds to PIP2, these results direct that PIP2 binds to the same motif that hyaluronan does in RHAMM.

### Figure 3

Binding of ³H-PLP2 to RHAMM in a transblot assay. ³H-PIP2 binds to RHAMM and this is competed with unlabeled PIP2.

### Figure 4

Rhamm localized in cytoplasm and nucleus.

### Figure 5

FITC-HA located in nucleus and to a lesser extent in cytoplasm.

### DETAILED DESCRIPTION OF EMBODIMENTS

### Methodology

### Figure 1

Bacteriophage containing the sequence RSHKTRSHH were isolated by exposure to biotinylated hyaluronan which was captured with streptavidin-coated beads. These bacteriophage were then exposed to biotinylated hyaluronan in the presencef of 5-250 µg of PIP2 or to 100 µg unlabeled exogenous hyaluronan. The phage that were then captured on the biotinylated hyaluronan probe under these various conditions were streaked on agar plates and grown at 37°C for 48 hours. Plates were then photographed.

### Figure 2

RHAMM variant 2 was synthesized using the RHAMMv2 cDNA transofrmed into E. Coli bacteria as described in detail in Yang, B., Yang; B.L., Savani, R.C. and Turley, E.A. The EMBO Journal 13, 286-296 (1994). The RHAMM protein was electrophoresed in SDS-PAGE (see above reference) and transblotted onto nitrocellulose acetate. Biotinylated hyaluronan was then incubated with the membrane and the bound biotinylated hyaluronan was detected by chemiluminescence as described in the above reference. In another identical blot, the biotinylated hyaluronan was mixed with PIP2 (250 µg/ml) and the mixture was then incubated with the membrane containing RHAMM. The bound biotinylated hyaluronan was then detected using chemiluminescence as above.

### Figure 3

Tritium labeled PIP2 (Amersham, 50 µCI/10ml) was incubated with RHAMM that had been separated on a SDS-PAGE and transblotted onto a nitrocellulose membrane as described above. The radiolabeled PIP2 was incubated with RHAMM for 1 hour, washed, then exposed to KODAK autoradiographic film for 1 month. The film was then scanned with a Biorad densitometre. The blot was stripped in detergent then reprobed for RHAMM using polyclonal anti-RHAMM antibodies to check that the radiolabeled band coincided with the RHAMM protein.

### CALCULATION OF DOSE OF PIP2 MIMETIC

Dose range that was effective in competing hyaluronan: 5-250 µg/10ml Blood volume of a rat is approximately 15 ml. and an average rat weighs 250 g.

Therefore the amount of PIP2 used per rat is 25-375 µg/250 g or 100-1500 µg/kg or 1-1.5 mg. per kilogram of body weight.

An effective amount of a modified Adenovirus containing gene therapy (for example, GMCSF - an acronym for Growth in Marrow Colony Stimulating Factor) is prepared for administration in known manner by persons skilled in the art. This modified Adenovirus is meant, when administered, to attract the immune system to the tumour. Rather than administering in the manner as previously known, Applicant proposes to modify the Adenovirus containing gene therapy further with the antisense peptide herein to provide a further modified "virus coat" on the surface thereof which is administered to the patient. (Persons skilled in the art can readily accomplish same by known methods.)

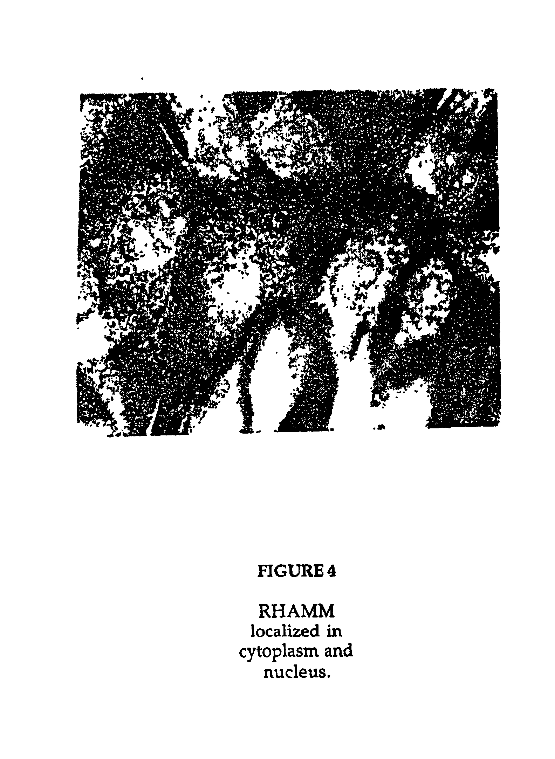

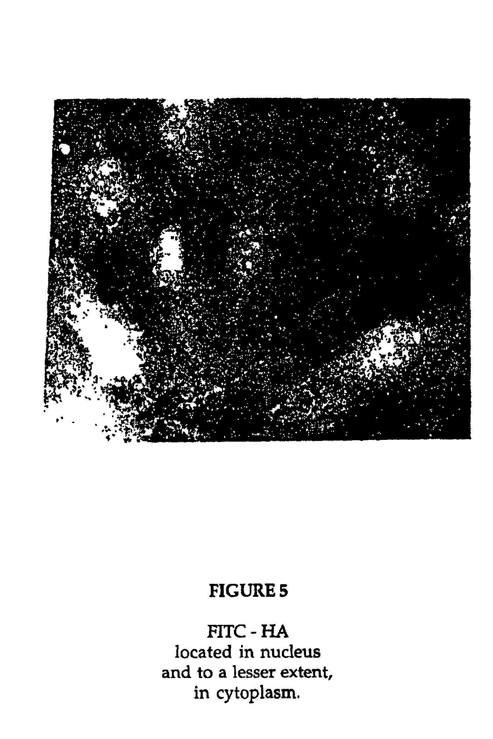

It has been demonstrated that HA (hyaluronan) receptors increase (are overly expressed) in Breast cancer, Pancreatic cancer, Colorectal cancer. High RHAMM expression (high HA receptor expression) occurs in Metastases. Thus, the administration of the Adenovirus modified to carry the anti-sense peptide (the mirror image of the binding domain) described above as an overcoat (and particularly anti-sense (b) above) will bind with RHAMM of the cancerous tumours and the Adenovirus will go right to the nucleus. The nucleus of the tumour will then be modified by the Adenovirus (efficiently making the gene product it has been designed to make) and the tumour is destroyed by the immune system. The basis for this proposal is that in a cell expressing RHAMM, Hyaluronan goes to the cell and binds with RHAMM, and about 15 minutes later, the hyaluronan has penetrated the nucleus. The anti-sense peptides for example, (b) antisense peptide, will therefore penetrate right into the cell and into the nucleus. Figures 4 and 5 are used to illustrate this action. Particularly, in Figure 5, FITC-HA (Hyaluronan) is located in the nucleus and in the cytoplasm. The same would occur with the anti-sense peptides discussed above. See "*Nuclear Localization Signals Direct Nuclear Proteins to the Nucleus* ", The Molecular Biology of the Cell, 3rd edition at pp. 563-564 (B. Albertz, D. Brag, J. Lewis, M. Raff, K. Roberts, J.O. Watson).

## Claims

1. Use of a binding agent (other than hyaluronan, a pharmaceutically acceptable salt thereof or other such forms of hyaluronic acid) selected from the group consisting of:
(i) 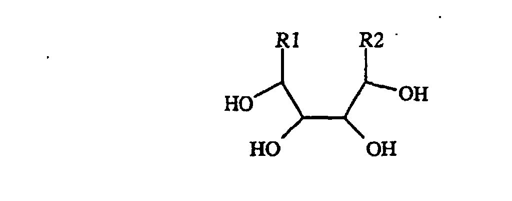 where either
(a) R₁ and R₂ form a bridge forming a closed ring molecule; or
(b) R₁ and R₂ are not joined and each of R₁ and R₂ is a lower alkyl
and
(ii) a binding agent which includes phosphoinositol-4,5-bisphosphate as an active portion,
in the manufacture of a medicament for the treatment of, or prevention of malignant tumours or restenosis in which the binding agent binds at the site of the disease or condition with at least one of the group of binding domains selected from:
(a) RSHKTRSHH
{*Single-Letter Code ofAmino Acids}*
also identified as: Arg-Ser-His-Lys-Thr-Arg-Ser-His-His {*Three-Letter Code ofAmino Acids*}
also encoded for by: CGC-TCG-CAC-AAG-ACC-AGG-TCG-CAC-CAC
(b) RPHFHKR
{*Single-Letter Code of Amino Acids}*
also identified as: Arg-Pro-His-Phe-His-Lys-Arg {*Three-Letter Code ofAmino Acids*}
also encoded for by:

CGG-CCC-CAC-TTC-CAC-AAG-CGG

2. Use according to claim 1 wherein the medicament comprises an effective amount of the binding agent, wherein the binding agent is mixed with the medicine and/or therapeutic agent.

3. Use according to claim 1 or claim 2 wherein the binding agent includes the active portion phosphoinositol-4,5-bisphosphate of PIP2.

4. Use according to any one of claims 1 to 3, wherein the amount of the binding agent is 0.1 to 1.5 mg per kilogram of body weight of the patient.

5. Use according to any one of claims 1 to 4, wherein the amount of the binding agent exceeds 0.2-1 mg per kilogram of body weight of the patient.

6. Use according to any one of claims 1 to 5, wherein the binding agent is selected from a derivative of combining PIP2 and a hydrophilic agent.

7. Use according to claim 6, wherein the hydrophilic agent is glucuronic acid.

8. Use according to any one of claims 1 to 7, wherein at least one phosphate moiety of the PIP2 active portion is substituted by a moiety selected from the group consisting of sulphate, carbonate, nitrate, sulphite or nitrite.

9. Use according to any one of claims 1 to 8, wherein the binding agent is selected from the compound: 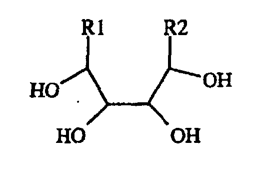 where either:
(a) R₁ and R₂ form a bridge forming a closed ring molecule; or
(b) R₁ and R₂ are not joined and each of R₁ and R₂ is a lower alkyl

10. Use according to any preceding claim wherein the binding agent is selected from the antisense peptide for:
(a) RSHKTRSHH
{*Single-Letter Code ofAmino Acids}* Arg-Ser-His-Lys-Thr-Arg-Ser-His-His {*Three-Letter Code of Amino Acids*}
(b) RPHFHKR
{*Single-Letter Code of Amino Acicls}* Arg-Pro-His-Phe-His-Lys-Arg {*Three-Letter Code of Amino Acids*}.

## Patentansprüche

1. Verwendung eines Bindemittels (außer Hyaluronan, einem pharmazeutisch akzeptablen Salz davon oder anderen solchen Formen der Hyaluronsäure), ausgewählt aus der Gruppe, die besteht aus: 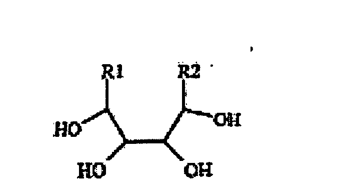
(i)
wobei entweder
(a) R₁ und R₂ eine Brücke bilden, welche ein Molekül mit geschlossenem Ring ausbildet; oder
(b) R₁ und R₂ nicht verbunden sind und jeweils R₁ und R₂ ein niedriges Alkyl sind und
(ii) einem Bindemittel, das Phosphoinositol-4,5-bisphosphat als einen aktiven Teil enthält,
bei der Herstellung eines Medikaments zur Behandlung von oder Vorbeugung gegen bösartige Tumore oder Restenose, wobei das Bindemittel an der Stelle der Krankheit oder des Leidens mit mindestens einer Bindungsdomäne aus der Gruppe von Bindungsdomänen bindet, die ausgewählt wird aus:
(a) RSHKTRSHH
(Einbuchstabencode für Aminosäuren)
auch identifiziert als: Arg-Ser-His-Lys-Thr-Arg-Ser-His-His (Dreibuchstabencode für Aminosäuren)
auch codiert durch: CGC-TCG-CAC-AAG-ACC-AGG-TCG-CAC-CAC
(b) RPHFHKR
(Einbuchstabencode für Aminosäuren)
auch identifiziert als: Arg-Pro-His-Phe-His-Lys-Arg (Dreibuchstabencode für Aminosäuren)
auch codiert durch:

CGG-CCC-CAC-TTC-CAC-AAG-CGG

2. Verwendung nach Anspruch 1, bei welcher das Medikament eine wirksame Menge des Bindemittels aufweist, wobei das Bindemittel mit der Medizin und/oder dem therapeutischen Mittel gemischt ist.

3. Verwendung nach Anspruch 1 oder 2, bei welcher das Bindemittel den aktiven Anteil Phosphoinositol-4,5-bisphosphat von PIP2 enthält.

4. Verwendung nach einem der Ansprüche 1 bis 3, bei welcher die Menge des Bindemittels bei 0,1 bis 1,5 mg pro Kilogramm Körpergewicht des Patienten beträgt.

5. Verwendung nach einem der Ansprüche 1 bis 4, bei welcher die Menge des Bindemittels 0,2 - 1 mg pro Kilogramm Körpergewicht des Patienten übersteigt.

6. Verwendung nach einem der Ansprüche 1 bis 5, bei welcher das Bindemittel aus einem Derivat aus der Kombination von PIP2 und einem hydrophilen Mittel ausgewählt ist.

7. Verwendung nach Anspruch 6, bei welcher das hydrophile Mittel Glucoronsäure ist.

8. Verwendung nach einem der Ansprüche 1 bis 7, bei welcher mindestens ein Phosphatanteil des PIP2 aktiven Teils durch einen Anteil ersetzt ist, der aus der Gruppe ausgewählt ist, die aus Sulfat, Carbonat, Nitrat, Sulfit oder Nitrit besteht.

9. Verwendung nach einem der Ansprüche 1 bis 8, wobei das Bindemittel ausgewählt wird aus der Verbindung: 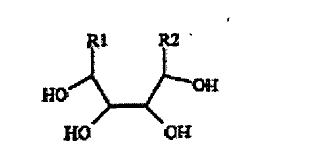 wobei entweder
(a) R₁ und R₂ eine Brücke bilden, welche ein Molekül mit geschlossenem Ring ausbildet; oder
(b) R₁ und R₂ nicht verbunden sind und jeweils R₁ und R₂ ein niedriges Alkyl sind

10. Verwendung nach einem der vorhergehenden Ansprüche, wobei das Bindemittel aus dem Antisense-Peptid für
(a) RSHKTRSHH
(Einbuchstabencode für Aminosäuren) Arg-Ser-His-Lys-Thr-Arg-Ser-His-His (Dreibuchstabencode für Aminosäuren)
(b) RPHFHKR
(Einbuchstabencode für Aminosäuren) Arg-Pro-His-Phe-His-Lys-Arg (Dreibuchstabencode für Aminosäuren)
ausgewählt ist.

## Revendications

1. Utilisation d'un agent de liaison (différent de l'hyaluronane, d'un sel pharmaceutiquement acceptable de celui-ci ou d'autres formes de ce type de l'acide hyaluronique) sélectionné parmi le groupe comprenant :
(i) 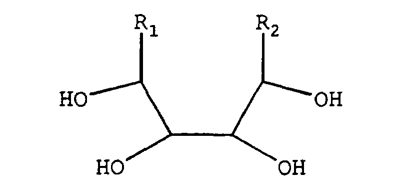 où soit
(a) R₁ et R₂ forment un pont formant une molécule cyclique fermée; ou
(b) R₁ et R₂ ne sont pas joints et chacun parmi R₁ et R₂ est un alkyle inférieur et
(ii) un agent de liaison qui comprend du phosphoinositol-4,5-bisphosphate comme partie active, dans la fabrication d'un médicament pour le traitement ou la prévention de tumeurs malignes ou d'une resténose dans lequel l'agent de liaison se lie au niveau du site de la maladie ou de l'état à au moins un parmi le groupe des domaines de liaison sélectionnés parmi :
(a) RSHKTRSHH
*{code à une lettre d'acides aminés}*
également identifié comme : Arg-Ser-His-Lys-Thr-Arg-Ser-His-His *(code à trois lettres d'acides aminés}*
également codé par : CGC-TCG-CAC-AAG-ACC-AGG-TCG-CAC-CAC
(b) RPHFHKR
*{code à une lettre d'acides aminés}*
également identifié comme : Arg-Pro-His-Phe-His-Lys-Arg *{code à trois lettres d'acides aminés}*
également codé par :

CGG-CCC-CAC-TTC-CAC-AAG-CGG

2. Utilisation suivant la revendication 1, dans laquelle le médicament comprend une quantité efficace de l'agent de liaison, dans laquelle l'agent de liaison est mélangé avec le médicament et/ou l'agent thérapeutique.

3. Utilisation suivant la revendication 1 ou la revendication 2, dans laquelle l'agent de liaison comprend la partie active phosphoinositol-4,5-bisphosphate ou PIP2.

4. Utilisation suivant l'une quelconque des revendications 1 à 3, dans laquelle la quantité d'agent de liaison est de 0,1 à 1, 5 mg par kilogramme de poids corporel du patient.

5. Utilisation suivant l'une quelconque des revendications 1 à 4, dans laquelle la quantité d'agent de liaison dépasse 0,2-1 mg par kilogramme de poids corporel du patient.

6. Utilisation suivant l'une quelconque des revendications 1 à 5, dans laquelle l'agent de liaison est sélectionné parmi un dérivé combinant PIP2 et un agent hydrophile.

7. Utilisation suivant la revendication 6, dans laquelle l'agent hydrophile est de l'acide glucuronique.

8. Utilisation suivant l'une quelconque des revendications 1 à 7, dans laquelle au moins une partie phosphate de la partie active PIP2 est remplacée par une partie sélectionnée parmi le groupe comprenant du sulfate, du carbonate, du nitrate, du sulfite ou du nitrite.

9. Utilisation suivant l'une quelconque des revendications 1 à 8, dans laquelle l'agent de liaison est sélectionné parmi le composé : 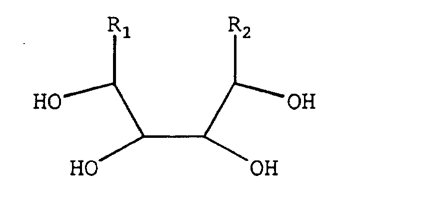 où soit
(a) R₁ et R₂ forment un pont formant une molécule cyclique fermée; ou
(b) R₁ et R₂ ne sont pas joints et chacun parmi R₁ et R₂ est un alkyle inférieur.

10. Utilisation suivant l'une quelconque des revendications précédentes, dans laquelle l'agent de liaison est sélectionné parmi le peptide anti-sens de :
(a) RSHKTRSHH
*{code à une lettre d'acides aminés}* Arg-Ser-His-Lys-Thr-Arg-Ser-His-His *{code à trois lettres d'acides aminés}*
(b) RPHFHKR
*{code à une lettre d'acides aminés}* Arg-Pro-His-Phe-His-Lys-Arg *{code à trois lettres d'acides aminés}*.
